(19) Europäisches Patentamt
European Patent Office
Office européen des brevets

(11) EP 1 455 808 B1

(12) **EUROPÄISCHE PATENTSCHRIFT**

(45) Veröffentlichungstag und Bekanntmachung des Hinweises auf die Patenterteilung:
**11.07.2007 Patentblatt 2007/28**

(21) Anmeldenummer: **02758095.0**

(22) Anmeldetag: **13.07.2002**

(51) Int Cl.:
***C07K 7/02*** *(2006.01)*
***A61K 38/08*** *(2006.01)*
***A61P 43/00*** *(2006.01)*
***A61P 39/00*** *(2006.01)*
***A61P 9/00*** *(2006.01)*
***A61P 11/00*** *(2006.01)*
***A61P 17/02*** *(2006.01)*
***A61P 1/16*** *(2006.01)*

(86) Internationale Anmeldenummer:
**PCT/DE2002/002583**

(87) Internationale Veröffentlichungsnummer:
**WO 2003/007980 (30.01.2003 Gazette 2003/05)**

(54) **PHOSPHINAT-PEPTIDANALOGA ALS INHIBITOREN DER PROCOLLAGEN-C-PROTEINASE (PCP) ZUR BEHANDLUNG VON FIBROTISCHEN ERKRANKUNGEN**

PHOSPHINATE-PEPTIDE ANALOGUES AS INHIBITORS OF PROCOLLAGEN-C-PROTEINASE (PCP) FOR TREATING FIBROTIC DISEASES

ANALOGUES PEPTIDIQUES DE PHOSPHINATE EN TANT QU'INHIBITEURS DE LA PROCOLLAGENE-C-PROTEINASE (PCP) POUR LE TRAITEMENT D'AFFECTIONS FIBROTIQUES

(84) Benannte Vertragsstaaten:
**AT BE BG CH CY CZ DE DK EE ES FI FR GB GR IE IT LI LU MC NL PT SE SK TR**

(30) Priorität: **14.07.2001 DE 10134243**

(43) Veröffentlichungstag der Anmeldung:
**15.09.2004 Patentblatt 2004/38**

(73) Patentinhaber:
- **Burchardt, Elmar R.**
  **42113 Wuppertal (DE)**
- **Stöcker, Walter**
  **48301 Nottulm (DE)**

(72) Erfinder:
- **Burchardt, Elmar R.**
  **42113 Wuppertal (DE)**
- **Stöcker, Walter**
  **48301 Nottulm (DE)**

(56) Entgegenhaltungen:
**DE-A- 19 850 072**

- **YIALLOUROS I ET AL: "Phosphinic peptides, the first potent inhibitors of astacin, behave as extremely slow-binding inhibitors." THE BIOCHEMICAL JOURNAL. ENGLAND 15 APR 1998, Bd. 331 ( Pt 2), 15. April 1998 (1998-04-15), Seiten 375-379, XP002219767 ISSN: 0264-6021 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Verwendung von Phosphinat-Peptidanaloga als Inhibitoren der Procollagen-C-Proteinase (PCP) zur Behandlung fibrotischer Erkrankungen. Die Erfindung auf dem Gebiet der Biotechnologie ist dadurch gekennzeichnet, dass geeignete Phosphinat-Peptidanaloga ebenso wie deren Salze entwickelt wurden, die spezifisch die Procollagen-C-Proteinase (PCP), ein Schlüsselenzym der Fibrogenese, inhibieren.

Es ist bekannt, dass die PCP ist ein Schlüsselenzym der Fibrogenese ist. Sie katalysiert die hydrolytische Abspaltung der Procollagenpropeptide von den Procollagenen I, II, II und IV sowie Laminin V[1]. Somit ist die PCP ein Schlüsselenzym der Collagenprozessierung[2]. In BMP-I-knock-out-Mäusen wurde nachgewiesen, dass eine komplette Abwesenheit der PCP zu einer unvollständigen Collagenprozessierung mit der Ablagerung von atypischen, lockeren Collagenfibrillen führt[3].

Die PCP ist wahrscheinlich auch für die hydrolytische Abspaltung der Propeptidsequenz der Lysyloxidase verantwortlich. Wahrscheinlich führt die Abspaltung der Prosequenz zur Aktivierung der katalytischen Lysyloxidaseaktivität der maturen Form[4]. Aktive Lysyloxidase verknüpft gegenüberliegende Collagenfibrillen kovalent miteinander. Auf diese Weise wird die biologische Stabilität des Collagens gegenüber dem Abbau durch Collagenasen indirekt auch durch die PCP erhöht.

Die PCP oder eng verwandte Proteine scheinen auch bei der Freisetzung von TGFβ-artigen Wachstumsfaktoren eine Rolle zu spielen. Durch neue Arbeiten konnte gezeigt werden, dass PCP-ähnliche Proteasen TGFβ-artige Wachstumsfaktoren aus einem inaktiven Komplex mit TGFß-Bindungsproteinen herauslösen können[56]. Dabei wird der Bindungspartner der TGFβ-artigen Wachstumsfaktoren durch spezifische Proteolyse zersetzt. Indirekt besitzt die PCP also möglicherweise auch eine TGFß-agonistische Aktivität. Daher kann der PCP eine entscheidende Rolle in der Fibrogenese zugesprochen werden.

Die PCP-Aktivität geht auf Splicevarianten des BMP-I-Gens zurück[789]. Bislang wurden verschiedene Splicevarianten dieses Proteins identifiziert, deren biologische Funktion zum Teil unklar ist. Bislang ist gesichert, dass die Splicevarianten BMP I-I und BMP I-III (tld-Variante) Procollagen und Pro-Lysyloxidase spezifisch schneiden können. Durch neuere Arbeiten wurden weitere BMP-1-Splicevarianten identifiziert, deren biologische Funktion und Substratspezifität aber noch ungeklärt ist.[10]

Obwohl die Expressionsklonierung und Aufreinigung der PCP bisher nur in kleinen Ausbeuten gelungen ist, sind von dem Enzym zahlreiche strukturelle Details bekannt. Die PCP gehört nämlich zur Familie der Astacinproteasen, und die Kristallstruktur dieses Proteins ist im Detail bekannt. Zwischen der katalytischen Domäne von BMP-I und Astacin gibt es einen sehr hohen Grad an struktureller Homologie, so dass es möglich war, vielen Aminosäuren der PCP-Proteasedomäne aufgrund dieser Homologie die wahrscheinliche strukturelle und biochemische Funktion zuzuordnen. "

Durch computergesteuertes "Molecular Modelling" konnte in der Vergangenheit die Substratbindung an das aktive Zentrum des BMP-I im molekularen Detail hergeleitet werden[12]. Diese Arbeiten führten zum "rational design" von Phosphinat-Peptidanaloga, die das Astacin mit hoher Wirkpotenz inhibieren. Der Komplex zwischen einem Phosphinatinhibitor und Astacin wurde strukturell aufgeklärt[13].

Bisher wurde trotz der hohen strukturellen Homologie zwischen Astacin und der katalytischen Domäne von BMP-1 davon ausgegangen, dass die beiden Proteasen sich aufgrund biochemischer Unterschiede hinsichtlich ihres Reaktionsverhaltens auch in der Hemmbarkeit durch Proteaseinhibitoren deutlich unterscheiden. Biochemische Unterschiede zwischen Astacin und BMP-I bestehen z.B. hinsichtlich der Substratspezifität (Astacin hydrolysiert als Verdauungsenzym des Flußkrebses relativ unspezifisch kollagenartige Proteine, während die PCP hochspezifisch an nur einer Stelle im Procollagenmolekül und in der Pro-Lysyloxidase schneidet).

In der Literatur sind bisher nur niederpotente Inhibitoren der PCP beschrieben, denen eine antifibrotische Wirkung zugeschrieben wird[14]. In einer neueren offengelegten Patenschrift werden C-terminal veresterte Derivate der hier beanspruchten Substanzen beschrieben[15]. Die direkt inhibierende Wirkung der freien Säure ist jedch aufgrund der unterschiedlichen Ladungseigenschaften der Moleküle bei physiologischem pH völlig überraschend.

Überraschenderweise wurde jetzt gefunden, dass Phosphinatpeptidanaloga der allgemeinen Formel (I)

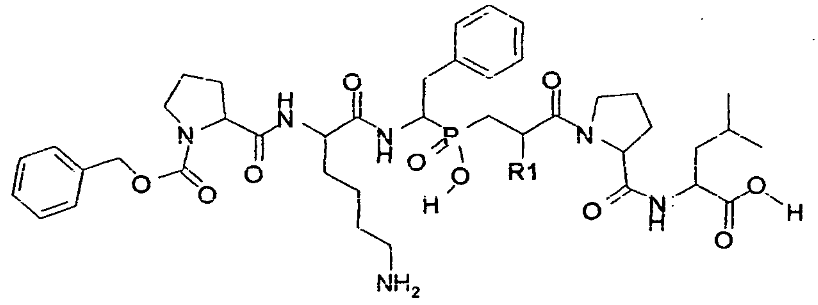

in welcher
R1 für Wasserstoff oder Methyl steht,
und deren Salze und Isomere die PCP mit sehr hoher Wirkpotenz inhibieren und deshalb zur Behandlung und Prophylaxe von fibrotischen Erkrankungen verwendet werden können.

Im Rahmen der Erfindung sind physiologisch unbedenkliche Salze bevorzugt. Physiologisch unbedenkliche Salze sind im allgemeinen Salze der erfindungsgemäßen Verbindungen mit anorganischen oder organischen Säuren. Bevorzugt werden Salze mit anorganischen Säuren, wie Salzsäure, Bromwasserstoffsäure, Phosphorsäure oder Schwefelsäure oder Salze mit organischen Carbon- oder Sulfonsäuren, wie Essigsäure, Maleinsäure, Fumarsäure, Apfelsäure, Zitronensäure, Weinsäure, Michsäure, Benzoesäure, oder Methansulfonsäure, Ethansulfonsäure, Phenylsulfonsäure, Toluolsulfonsäure oder Naphtalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racematformen sowie die Diastereomerengemische.

Die Verbindungen der allgemeinen Formel (I) können in allen enantiomeren und diastereomeren Formen vorliegen. Bevorzugt sind diejenigen Isomeren, in denen die aus Prolin, Lysin und Leucin gebildeten Molekülteile die L-Konfiguration besitzen, ebenso wie deren Salze

Besonders bevorzugt werden diejenigen Phospinatpeptidanaloga der allg. Formel (Ia), die wie folgt konfiguriert sind

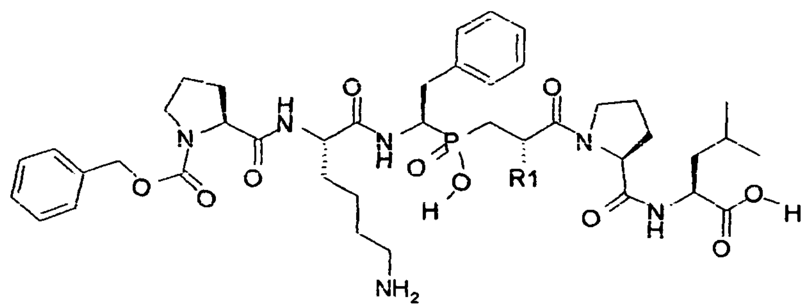

in welcher
R1 für Wasserstoff oder Methyl steht
und deren Salz und Isomere zur Behandlung und Prophylaxe von fibrotischen Erkrankungen verwendet.

Ganz besonders bevorzugt wird die Verbindung der Formel (Ib)

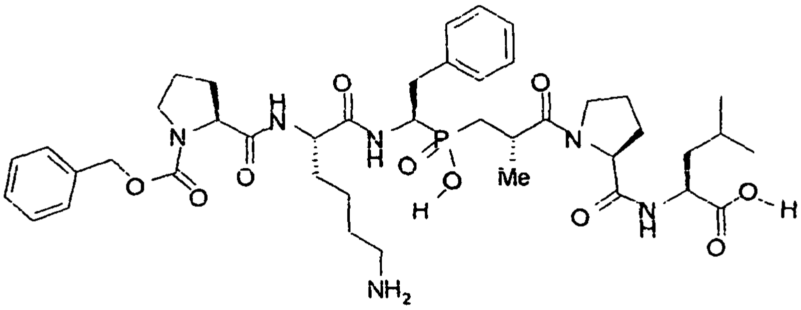

,

im folgenden als **Z---PKF(PC)APL-O-H** bezeichnet,
deren Enantiomere und deren Salze zur Behandlung und Prophylaxe von fibrotischen Erkrankungen verwendet.

Die Verbindungen der allgemeinen Formel (I) sind bekannt und können nach üblichen Methoden der Peptidsynthese aus entsprechenden Phinioylverbindungen hergestellt werden[16 17].

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formeln (I) bestehen, sowie Verfahren zur Herstellung dieser Zubereitungen.

Die Wirkstoffe der Formeln (I) sollen in diesen Zubereitungen in einer Konzentration von 00,1 bis 99,5 Gew.-%, bevorzugt von 0,1 bis 95 Gew.-%, der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Individuums, vom individuellen Verhalten gegenüber dem Medikament, der Art und der Schwere der Erkrankung, der Art der Zubereitung und Applikation, sowie dem Zeitpunkt bzw. Intervall, zu welchem die Verabreichung erfolgt.

Diese Ergebnisse waren unerwartet, weil sich BMP-I, Meprin und Astacin vor allem im sogenannten SI'-Loop unterscheiden. Ein Ausschnitt aus diesem Loop ist hier dargestellt:

| | |
|---|---|
| Astacin: | T--DPYD |
| BMP-I: | KPPIG-Q |
| Meprin: | I -- IG-Q |

Dieser Loop bildet den essentiellen Teil der SI'-Bindungstasche der Astaein-ähnlichen Proteasen. Hier liegt, wie am Beispiel des Astacins und des Meprins gezeigt wurde, der Schlüssel zur unterschiedlichen Substrat- und Inhibitorspezifität beider Enzyme. In der SI'-Tasche unterscheiden sich Meprin und Astacin sehr deutlich. Die PCP hat zusätzlich zwei Prolinreste und einen Lysinrest in dieser Region. Wie durch Computermodelling der Proteasedomäne der PCP gezeigt werden konnte, ist der Lysinrest sehr wahrscheinlich an der Bindung der Carboxylgruppe in der Seitenkette des Aspartats in P1' neben der Spaltstelle im Procollagen beteiligt. Die Prolinreste liegen nach dem Modell in der cis-Konfiguration vor und sind eine Eigentümlichkeit der BNT-I-Subfamilie unter den Astacinen. Im Gegensatz dazu spaltet Astacin nicht neben sauren Resten. Aus diesen Gründen war es völlig unerwartet, dass sich ein für die Inhibition von Astacin entworfenes Phosphinat[18] als effektiver PCP-Hemmstoff erwies.

Die erfindungsgemäßen Verbindungen der allgemeinen Formel (I) zeigen ein nicht vorhersehbares, wertvolles pharmakologisches Wirkspektrum.

Überraschenderweise zeichnen sich die erfindungsgemäßen Verbindungen durch eine sehr hohe Wirkpotenz bei der Inhibition der PCP aus.

Die erfindungsgemäßen Verbindungen sind deshalb zur Behandlung von Leberfibrosen jeder Genese und von Fibrosen mit anderer Organmanifestation geeignet.

Dazu gehört eine diverse Gruppe von Krankheiten, die mit einer qualitativ veränderten Collagenproduktion

oder mit einer verstärkten Ablagerung von Collagen im Extrazellularraum einhergehen. Zu dieser Gruppe von Erkrankungen gehören Leberfibrosen unterschiedlichen Ursprungs, wie z.B. alkoholische Leberzirrhose, biliäre Zirrhose, Hepatitiden viraler oder anderer Genese, idiopathische interstitielle Fibrosen, idiopathische Lungenfibrosen, akute pulmonale Fibrosen, das "acute respiratory distress syndrome" (ARDS), perimuskuläre Fibrosen, perizentrale Fibrosen, Dermatofibrome, Nierenfibrosen, die diabetische Nephropathie, Glomerulonephritiden, die systemische oder lokale Sklerodermie, Keloide, die hypertrophe Narbenbildung, Gelenkadhäsionen, Arthrosen, Myelofibrosen, Vernarbungen der Cornea, die cystische Fibrose, muskuläre Fibrosen, die Duchenne'sche Muskeldystrophie, Ösophagusstrikturen, Morbus Ormond, Morbus Crohn, Colitis ulcerosa und Aneurysmen der großen Gefäße.

Außerdem umfasst die Erfindung fibrotische Erkrankungen, die initiiert oder hervorgerufen werden durch chirurgische Narbenrevisionen, plastische Chirurgie, Glaukome, Kataraktfibrosen, Vernarbungen der Cornea, die sogenannte "graft versus host disease", chirurgische Eingriffe an Sehnen, Nerveneinklemmungssyndrome, die Dupuytren'sche Kontraktur, Adhäsionen infolge gynäkologischer Eingriffe, pelvische Adhäsionen, peridurale Fibrosen, Erkrankungen der Schilddrüse oder der Nebenschilddrüsen, durch metastatischen Knochenbefall, durch das multiple Myelom oder durch Restenosen.

Nachweis der PCP-Aktivität im Fluoreszenzdequenchtest

Zum Nachweis der PCP-Aktivität wurde ein synthetisches Dekapeptidsubstrat mit der Sequenz DABCYL - Asp - Phe - Tyr - Arg - Ala - Asp - Gen - Pro - Arg - Asp (EDANS) - $NH_2$ durch Zusatz von PCP gespalten. Diese Peptidfrequenz entspricht der Region im Procollagen $\alpha_2$ (I), die von PCP gespalten wird. Die Spaltsequenz ist biochemischen Experten bekannt[19], ebenso das Verfahren des Dequenchtests[20].

Die Konzentration des synthetischen Peptids war 5,6 μM, die finalen Pufferbedingungen waren: 50 mM Tris pH 7,5, 150 mM NaCl und 0,005% Brij35.

Die Durchführung des kinetischen Test erfolgte wie im folgenden beschrieben: Das Fluoreszenzsubstrat wurde in 90 μl Reaktionspuffer gelöst. Die Kinetik des Substratumsatzes durch MMP-2 wurde in Doppelbestimmungen durch Fluoreszenzmessung (Ex. 365 nm/Em. 450 nm) zwischen 0 bis 120 min detektiert. Die Reaktion wurde durch Zugabe einer geeigneten Aktivität von PCP, in 10 μl Reaktionspuffer gelöst, gestartet. In Zeitintervallen von anfangs 5min und später in größeren Zeitintervallen wurde die Emission als ein Maß der proteolytischen Aktivität gemessen. Als Negativkontrolle wurde der spontane Zerfall des Fluoreszenzsubstrats bei Zugabe von Reaktionspuffer ohne PCP-Aktivität gemessen. Die Fluoreszenzmessungen erfolgten über einen Zeitraum von ca. 4h bei 37°C. Nach Ablauf der Reaktion wurde das Substrat durch Zugabe von Proteinase K (Boehringer Mannheim, 1,44μg/Reaktionsansatz, gelöst in 10μl PBS) und Inkubation bei 37°C für 20 min vollständig zersetzt. Die Vollständigkeit der Hydrolyse wurde dadurch gezeigt, dass keine Zunahme der Fluoreszenz in Abhängigkeit von der Zeit mehr erfolgte.

Der relative Substratumsatz in % der Gesamtmenge errechnete sich aus:

$$\% \text{ Umsatz} = (F_t - F_{-t}) \div (F_{total} - F_{ini}) \times 100\%$$

wobei $F_t$ die relative Fluoreszenz nach einem Zeitintervall t unter Inkubation mit PCP ist. $F_{-t}$ ist die korrespondierende relative Fluoreszenz nach dem Zeitintervall t ohne PCP-Zusatz, $F_{total}$ ist die Fluoreszenz nach Totalhydrolyse durch Zusatz von Proteinase K, und $F_{ini}$ ist die initiale relative Fluoreszenz vor Start der Reaktion durch Zugabe von Proteinase K.

In dem Test wurde die verwendete PCP-Aktivität typischerweise so eingestellt, dass mit uninhibiertem Enzym innerhalb des Messzeitraumes ca. 20% des Substrats umgesetzt wurden.

In Abbildung 1 ist eine typische Reaktionskinetik (% Umsatz) unter Zugabe des Phospinatinhibitors Z---PKF (PC)APC---O-X dargestellt.

Die prozentuale Inhibition unter Zugabe der Inhibitoren errechnete sich nach:

$$\% \text{ Inhibition} = 100\% \times \% \text{ Umsatz (mit Inhibitor)} \div \% \text{ Umsatz (ohne Inhibitor)}$$

In Abbildung 2 wird die Konzentrations-Wirkungsbeziehung eines Phosphinatinhibitors dargestellt.

Rekombinante Herstellung der katalytischen Domäne von BMP-I

Im Fluoreszenzaktivitätstest wurde die katalytische Domäne von BMP-I eingesetzt. Die entsprechende cDNA-Sequenz ist aus der Literatur bekannt[21] und wurde durch RT-PCR erhalten. Die entsprechenden Primers enthielten

upstream eine *Ecl*XI und downstream eine SalI-Schnittstelle, so dass das Insert gemäß den Angaben des Herstellers Biometra[22] in den *E. coli*-Expressionsvektor pASK75 inseriert werden konnte. Dies geschah durch einen Verdau des Plasmids pASK75 mit den Restriktionsenzymen *Bsa*I und *Sal*I. Diese Schnittstellen sind Bestandteile der "multiple cloning site" von pASK75. Die Details dieser Klonierungsschritte sind molekularbiologischen Experten aus der Literatur bekannt[23].

Die Besonderheit dieses Clonierungsansatzes liegt darin, dass die Proteasedomäne ohne zwischengeschaltete Aminosäuren auf die Prosequenz des "outer membrane protein A" (ompA) folgt. Da die Prosequenz von ompA bei der Sekretion ins Periplasma proteolytisch abgespalten wird, wird bei erfolgreicher periplasmatischer Sekretion der N-Terminus der katalytischen Domäne von BMP-I freigelegt. Es ist bekannt, dass der N-Terminus bei Proteasen aus der Astacinfamilie eine besondere Rolle bei deren Aktivierung spielt. Er faltet sich nämlich ins aktive Zentrum des Enzyms zurück und bildet so über Wasserstoffbrückenbindungen die stabile aktive Konformation des Enzyms aus.

Die Expression der Proteasedomäne von BMP-I wird durch zwei Faktoren erschwert:

1. Das Protein neigt zur Bildung von schwer löslichen *inclusion bodies.* Dies zeigt sich z.B. dadurch, dass in den meisten *E. coli*-Stämmen bei der Expression dieses Proteins keine löslichen Formen gefunden wurden. Außerdem ist die Verweildauer des rekombinanten Proteins im Cytoplasma wahrscheinlich sehr kurz, so dass bei seiner Expression regelmäßig das N-terminale Methionin nicht abgespalten wird. Somit ist es häufig schwierig, den freien N-Terminus zu rekonstituieren, der für die Herstellung der enzymatische Aktivität der Proteasedomäne notwendig ist.

2. Das aktive Protein ist für *E. coli* toxisch, so dass die Expression von ATG-Vektoren in den meisten Stämmen zu schlechten oder überhaupt keinen Ausbeuten an rekombinantem Protein führt.

Bisher sind nur wenige Literaturstellen bekannt, in denen über die erfolgreiche rekombinante Expression des Proteins berichtet wird. Die beschriebenen Ausbeuten sind jeweils sehr gering. In Verbindung mit der niedrigen Wechselzahl der PCP (6 $h^{-1}$)[24] wird klar, weshalb bisher erst wenige potente Inhibitoren der PCP beschrieben wurden.

Für die Testung und Findung von PCP-Inhibitoren ist die Herstellung größerer Aktivitäten an PCP unerlässlich. Dieses Problem wurde mit dem oben beschriebenen Konstrukt gelöst. Durch die periplasmatische Sekretion des Proteins wird die cytoplasmatische Toxizität gemindert, zumal die PCP erst beim Übertritt ins Perisplasma durch die ompA-spaltende Protease aktiviert wird. Die exzessive inclusion body-Bildung kann durch Absenkung der Reaktionstemperatur gemäß den Empfehlungen aus dem Protokoll des Herstellers und durch die Auswahl eines geeigneten Expressionsstamms erreicht werden.

<u>Spezifitätsnachweis der PCP-Inhibitoren durch Untersuchung an der Matrix-Metalloprotease Typ 2 (MMP2) *in vitro*</u>

MMP-2 (72 kD Type IV Colagenase, Gelatinase A) gehört zur Familie der Matrixmetalloproteasen, die in der Lage sind, Bestandteile der extrazellulären Matrix (Elastin, Fibronectin, Kollagene Typ IV, VII und X in helikalen Domänen und Gelantine) abzubauen[25].

Das Enzym wird als inaktive Vorstufe (72 kD) von zahlreichen Zellen mesenchymalen Ursprungs sezerniert. Zur Bildung der aktiven 62 kD-Form wird *in vivo* ein aminoterminales Propeptid abgespalten. Im nachfolgend beschriebenen Enzymassay (*in vitro*) wurde durch p-Aminophenyl-Quecksilberacetat (APMA) die latente in die aktive Form überführt.

<u>Aktivierung:</u> 1 U MMP-2 (Boehringer Mannheim, isoliert aus humanen Fibrosarcoma-Zellen, 200 U/mg) wurde in 50 mM Tris/Cl pH 7,5, 0,05 % (v/v) Triton X 100, 1 mM $CaCl_2$ mit 2,5 mM APMA 30 Minuten bei 37°C aktiviert[26].

<u>Enzymassay:</u> In einem Volumen von 195 μL wurden 0,5 U aktivierte MMP-2 mit 1 μM Z-PKF(PC)APL --- 0-X 10 Minuten bei 37°C inkubiert. Durch Zugabe von 5 μl Substrat (final 50μM) ((Dnp-Fro-β-cyclohexyl-Ala-Gly-Cys (Me)-His-Ala-Lys(N-Me-Abz)-NH2), Bachem AG, wurde die enzymatische Reaktion gestartet[27]. Die Emmision wurde als ein Maß der protolytischen Enzymaktivität von MMP-2 durch Fluoreszenzmessung (Excitationswellenlänge 365 nm und initiale Emission bei 538 nm) in Zeitintervallen zwischen 0 bis 120 min detektiert. Die Spaltsequenz ist biochemischen Experten bekannt[28], ebenso das Verfahren des Dequenchtests[29].

Folgende Reaktionsansätze wurden durchgeführt: Als Negativkontrolle diente der spontane Zerfall des Substrates ohne MMP-2. Nicht aktivierte MMP-2, bzw. durch APMA aktivierte MMP-2, diente als Referenz zur aktivierten MMP-2 inkubiert mit 1 μM Z---PKF(PC)APL-O-H (Abbildung 2).

Abbildung 2 zeigt klar die Spezifität des hochwirksamen Phosphinat-Peptidanalogen Z-PKF(PC)APL---O-H zu PCB. Die verwendtete aktivierte Matrix-Metalloprotease Typ 2 wurde auch bei Konzentrationen bis zu 1 μM (dosisabhängig) nur unwesentlich bei der Umsetzung des fluoreszenzmarkierten Peptids inhibiert.

## Demonstration der biologischen Wirksamkeit

Die biologische Wirksamkeit der Substanzen kann in Zellkulturassays und *in vivo* demonstriert werden. Nach Applikation der Inhibitoren kann in humanen Zellinien z.B. der Abfall der Konzentration an freiem Procollagen alpha 1 Typ III Propeptid in den Überständen gemessen werden, weil dieses Peptid durch die Aktivität der PCP freigesetzt wird. Zur Messung der PIIICP-Konzentrationen im Überstand kann ein kürzlich etablierter Assay verwendet werden[30].

Zum Nachweis des antifibrotischen Effekts der Substanzen in der Leber können z.B. das Tiermodell der akuten[31] oder chronischen[32] Tetrachlorkohlenstoff-induzierten Leberschädigung, das Modell der Leberfibrose durch Gallengangsligatur[33] oder die durch heterologes Serum induzierte Leberfibrose[34] verwendet werden. Auch andere Tiermodelle, bei denen eine Leberfibrose auftritt, können zum Nachweis des antifibrotischen Effekts verwendet werden.

Je nach Organmanifestation oder Art der fibrotischen Schädigung können auch Tiermodelle für andere Fibrosemanifestationen, z.B. im Herzen, in den Nieren, in den Lungen, in der Haut oder anderen Organen verwendet werden.

Die Reduktion der Collagenablagerung kann z.B. durch die Bestimmung des Hydroxyprolingehalts[35] der fibrotischen Organe oder durch quantitative Morphometrie erfolgen[36].

## Beschreibung der Abbildungen

Abbildung 1 zeigt den Substratumsatz des DABCYL-EDANS-Dekapeptides durch rekombinante PCP in Abhängigkeit von der Zeit. Die Enzymaktivität wird in Anwesenheit von 100 nM Inhibitor fast vollständig inhibiert.

Abbildung 2 zeigt die Konzentrations-Wirkungsbeziehung des Phosphinatinhibitors Z-PKF(PC)APL---O-H auf PCP

Abbildung 3 zeigt den Substratumsatz eines MMP-2 proteasespezifischen fluoreszenzmarkierten Peptides durch Metalloproteinase Typ 2 in Abhängigkeit von der Zeit. Die MMP-2 Enzymaktivität wird in Anwesenheit von 1 μM Z---PKF(PC)APL---O-H nicht inhibiert.

a) Substrat: spontaner Zerfall von 50 μM Substrat in Testpuffer
b) APMA: APMA und 50 μM Substrat
c) MMP-2 inaktiv: MMP-2, nicht APMA-aktiviert, und 50 μM Substrat
d) MMP/APMA: MMP-2, APMA-aktiviert, und 50 μM Substrat
e) 1 μMZ---PKF(PC)APL---O-H:

MMP-2, APMA-aktiviert, und 50 μM Substrat
+ 1 μMZ---PKF(PC)APL---O-H

## Literatur

[1] Amano S, Takahara K; Gerecke D, Nishiyama T, Lee S, Greenspan DS, Burgeson RE (1996) Bone morphogenetic protein-1 is the processing enzyme for laminin 5 in human keratinocytes. Mol. Biol. Cell 7 (suppl.) 58A

[2]Olsen BJ (1996) Morphogenesis: collagen it takes and bone it makes. Curr. Biol. 6: 645-647

[3] Suzuki N, Labosky PA, Furata Y, Hargett I, Dunn R, Fogo AB, Takahara K, Peters DM, Greenspan DS, Hogan BL (1996) Failure of ventral body wall closure in mouse embryos lacking a procollagen C-proteinase encoded by BMP-1, a mammalian gene related to Drosophila tolloid. Development 122: 3587-3595

[4] Pachenko MV, Stetler-Stevenson WG, Trubetskoy OV, Gacheru SN, Kagan HM (1996) Metalloproteinase activity secreted by fibrogenic cells in the processing of prolyslyl oxidase. Potential role of procollagen C-proteinase. J. Biol. Chem. 2 71: 7113-7119

[5] Marques G, Musacchio M, Shimell MJ, Wünnenberg-Stapleton K, Cho KWY, O'Connor MB (1997) Production of a DPP activity gradient in the early drosophila embryo through the opposing actions of the SOG and TLD proteins. Cell 91: 417-426

[6] Blader P, Rastegar S, Fischer N, Strähle U (1997) Cleavage of the BMP antagonist chordin by zebrafish tolloid. Science 278: 1937-1940

[7]Kessier E, Takahara K, Biniaminow L, Brusel M, Greenspan DS (1996) Bone morphogenetic protein- 1: The type I procollagen C-proteinase

[8] Reddi AH (1996) BMP-1: Resurrection as procollagen C-proteinase. Science 217: 463

[9] Li SW, Sieron AL, Fertala A, Hojima Y, Arnold WV, Prockop DJ (1996) The C-proteinase that processes procollagens to fibrillar collagens is identical to the protein previosly identified as bone-morphogenetic protein-1. Proc. Natl. Acad.

Sci. 93: 5127-5130

[10] Janitz M, Heiser V, Böttcher U, Landt O, Lauster R (1998) Three alternatively spliced variants of the gene coding for the human bone morphogenetic protein-1. J. Mol. Med. 76: 141-146

[11] Stöcker W, Gomis-Rüth FX, Bode W, Zwilling R (1993) Implications of the three-dimensional structure of astacin for the structure and function of the astacin family of zincendopeptidases. Eur. J. Biochem. 214: 215-231

[12] Stöcker W, Grams F, Baumann U, Reinemer P, Gomis-Rüth FX, McKay DB, Bode W (1995) The metzincins - Topological and sequential relations between the astacins, adamalysins, serralysins, and matrixins (collagenases) define a superfamily of zincendopeptidases. Prot. Sci.: 823-840

[13] Grams F, Dive V, Yiotakis A, Yiallouros I, Vassilou S, Zwilling R, Bode W, Stöcker W (1996) Structure of astcin with transition-state analogue inhibitor. Nature Struct. Biol. 3: 671-675

[14] Brenner M, Ho WB (1996) C-proteinase inhibitors for the treatment of disorders related to the overproduction of collagen patent application WO 97/05865

[15] Burchardt ER, Schauer M, Stöcker W (1998)Phosphinat-Peptidanaloga zur behandlung von firotischen Erkrankungen. DE 198 50 072

[16] Yiotakis A, Vassilio S, Jiracek J, Dive V (1996) Protection of the Hydroxyphosphinyl Function ofPhosphinic Dipeptides by Adamantyl. Application to the Solid-Phase Synthesis of Phosphinic Peptides. J. Org. Chem. 61: 6601-6605

[17] Campagne JM, Coste J, Guillou L, Heitz A, Jouin P (1993) Solid phase synthesis of phosphinic peptides. Tetrahedron Lett. 34: 4181-4184

[18] Yiallouros I, Vassiliou S, Yiotakis A, Zwilling R, Stöcker W, Dive V (1998) Phosphinic peptides, the first potent inhibitors of astacin, behave as extremely slow-binding inhibitors. Biochem. J. 331:375-379

[19] Lee ST; Kessler E, Greenspan DS (1990) Analysis of site-directed mutations in human pro-a2(I) collagen which block cleavage by the C-proteinase. J. Biol. Chem. 265: 21992-21996

[20] Matayoshi ED, Wang GT, Krafft GA, Erickson J (1989) Novel fluorogenic substrates for assaying retroviral proteases by resonance energy transfer. Science 247 : 954-958

[21] JM, Rosen V, Celeste AJ, Mitsock LM, Whitters MJ, Kriz RW, Hewick RM, Wang EA (1988) Novel regulators ofbone formation: molecular clones and activities. Science 242: 1528-1534

[22] Instruction manual strep tag II System, Biometra 1994

[23] Sambrook J, Fritsch EF, Maniatis T (1989) Molecular Cloning. A Laboratory Manual. 2nd edition. Cold Spring Harbor Laboratory Press

[24] Prockop DJ, Sieron AL, Li SW (1998) Procollagen N-proteinase and procollagen C-proteinase. Two unusual metalloproteinases that are essential for procollagen processing probably have important roles in development and cell signaling. Matrix Biol. 16: 399-408

[25] Aimes, RT & Quigley JP (1995) Matrix Metalloptotenase-2 is an interstitial Collagenase-Inhibitor-free enzyme catalyzes the cleavage of collagen fibrils and soluble native type-I collagen generating the specific ¾-length and ¼-length fragments, J. Biol. Chem., 270: 5872-5876

[26] Gregory A. Grant, Arthur Z. Eiseb, Barry L. Marmer, William T. Roswit, and Gregory I. Goldberg (1987) The Activation of Human Fibroblast Procollagenase. J. Biol. Chem. 262: 5886-5889

[27] Bickett DM, Green MD, Berman J, Dezube M, Howe AS, Brown PJ, Roth JT, McGeehan GM (1993) A high throughput fuorogenic substrate for interstitial collagenase (MMP-1) and gelatinase (MMP-9). Anal. Biochem. 212: 58-64

[28] Lee ST; Kessler E, Greenspan DS (1990) Analysis of site-directed mutations in human pro-a2(I) collagen which block cleavage by the C-proteinase. J. Biol. Chem. 265: 21992-21996

[29] Matayoshi ED, Wang GT, Krafft GA, Erickson J (1989) Novel fluorogenic substrates for assaying retroviral proteases by resonance energy transfer. Science 247: 954-958

[30] Burchardt ER, Schröder W, Heke M, Kohlmeyer J, Neumann R, Kroll W (1997) Expression cloning of C-terminal procollagen (III) propeptide and its use in a novel serum assay to monitor liver fibrogenesis. Hepatology 26: 487A

[31]Johnson S J, Hines JE, Burt AD. Phenotypic modulation of perisinusoidal cells following acute liver injury: a quantitative analysis. Int. J. Exp. Path. 1992; 73: 765-772

[32] McLean E, McLean A, Sutton P. Instant Cirrhosis. An improved method for producing cirrhosis of the liver in rats by simultaneous administration of carbon tetrachloride and phenobarbitone. Br. J. Exp. Pathol. 1969; 50: 502-506

[33] Kountouras J, Billing B, Scheuer P. Prolonged bile obstruction: a new experimental model for cirrhosis in the rat. Br. J. Exp. Pathol. 1984; 65: 305-311

[34] Bhunchet E, Wake K. Suppression of experimental hepatic fibrosis by administration of vitamin A.Lab. Invest. 1985; 52: 182-194

[35] Gerling B, Becker M, Waldschmidt J, Rehmann M, Schuppan D. Elevated serum aminoterminal procollagen type-III-peptide parallels collagen accumulation in rats with secondary biliary fibrosis. Hepatology 1996; 25: 79-84

[36] Kauschke SG, Knorr A, Olzen M, Burchardt ER (1997) Expression of collagen (III) as determined by quantitative

PCR and its correlation with extracellular collagen deposition in the rat CCl4 model of liver fibrosis. Hepatology 26: 538A

**Patentansprüche**

1. Phospinat-Peptidanaloga der allgemeinen Formel (II)

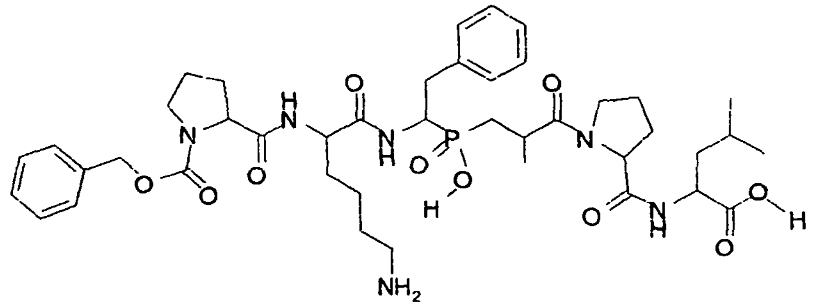

und/ oder deren Stereoisomere und Salze.

2. Verbindungen gemäß Anspruch 1, **dadurch gekennzeichnet, dass** Verbindungen der allgemeinen Formel (II) mit der in der Formel (Ib) aufgezeigten Konfiguration

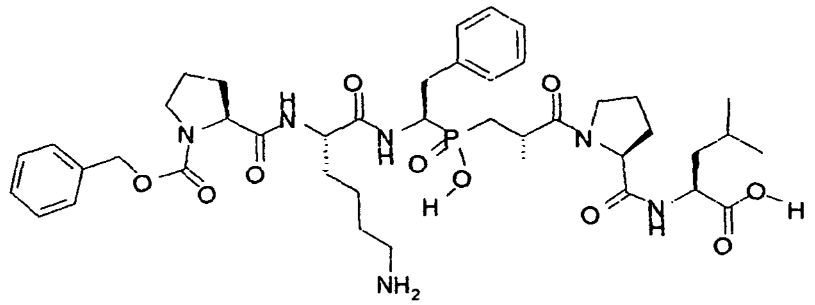

und/oder deren Salze ausgewählt sind.

3. Verbindungen der Formel I

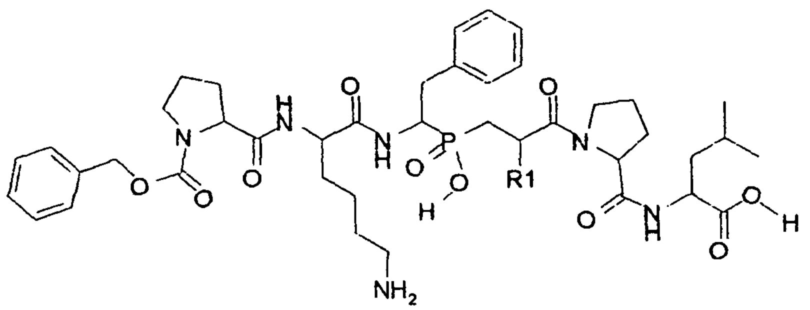

in welcher
R1 für Wasserstoff oder Methyl steht,
und/ oder deren Stereoisomere und Salze zur Verwendung als Arzneimittel zur Prophylaxe und/oder Behandlung von Erkrankungen.

4. Arzneimittel, enthaltend mindestens eine Verbindung der Formel I, wie in Anspruch 3 definiert, und mindestens einen weiteren Hilfsstoff.

5. Arzneimittel, enthaltend mindestens eine Verbindung der Formel I, wie in Anspruch 4 definiert, und mindestens einen weiteren Wirkstoff.

6. Verwendung mindestens einer Verbindung der Formel (I), wie in Anspruch 3 definiert, zur Herstellung eines Arzneimittels zur Prophylaxe und/oder Behandlung von fibrotischen Erkrankungen wie u.a. Leberfibrosen unterschiedlichen Ursprungs, wie z.B. alkoholische Leberzirrhose, biliäre Zirrhose, Hepatitiden viraler oder anderer Genese, idiopathische interstitielle Fibrosen, idiopathische Lungenfibrosen, akute pulmonale Fibrosen, das "acute respiratory distress syndrome" (ARDS), perimuskuläre Fibrosen, perizentrale Fibrosen, Dermatofibrome, Nierenfibrosen, die diabetische Nephropathie, Glomerulonephritiden, die systemische oder lokale Sklerodermie, Keloide, die hypertrophe Narbenbildung, Gelenkadhäsionen, Arthrosen, Myelofibrosen, Vernarbungen der Cornea, die cystische Fibrose, muskuläre Fibrosen, die Duchenne'sche Muskeldystrophie, Ösophagusstrikturen, Morbus Ormond, Morbus Crohn, Colitis ulcerosa und Aneurysmen der großen Gefäße sowie fibrotische Erkrankungen, die initiiert oder hervorgerufen werden durch chirurgische Narbenrevisionen, plastische Chirurgie, Glaukome, Kataraktfibrosen, Vernarbungen der Cornea, die sogenannte "graft versus host disease", chirurgische Eingriffe an Sehnen, Nerveneinklemmungssyndrome, die Dupuytren'sche Kontraktur, Adhäsionen infolge gynäkologischer Eingriffe, pelvische Adhäsionen, peridurale Fibrosen, Erkrankungen der Schilddrüse oder der Nebenschilddrüsen, durch metastatischen Knochenbefall, durch das multiple Myelom oder durch Restenosen.

## Claims

1. Phosphinate-peptide analogs of the general formula (II)

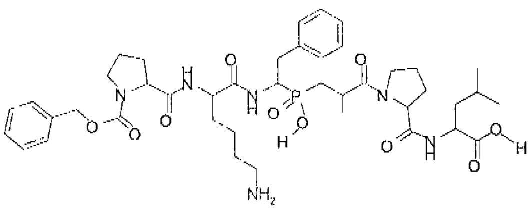

,

and/or their stereoisomers and salts.

2. Compounds according to Claim 1, **characterized in that** compounds of the general formula (II) having the configuration shown in formula (Ib)

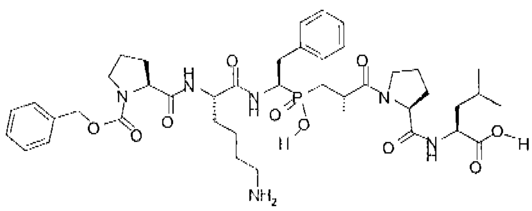

and/or their sails are selected.

3. Compounds of the formula (I)

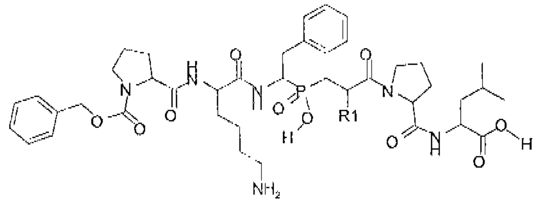

in which
$R^1$ represents hydrogen or methyl,
and/or their stereoisomers and salts for use as drugs for the prophylaxis and/or treatment of diseases
and/or its enantiomers and salts is/are used.

**4.** Drugs containing at least one compound of formula (I), as defined in Claim 3, and at least one additional excepient.

**5.** Drugs containing at least one compound of formula (I), as defined in Claim 4, and at least one additional active ingredient.

**6.** Use of at least one of the compounds of formula (I), as defined in Claim 3, for the preparation of drugs for the prophylaxis and/or the treatment of fibrotic diseases, such as liver fibroses of differing origin, such as alcoholic liver cirrhosis, biliary cirrhosis, hepatitis of viral or other genesis, idiopathic interstitial fibroses, idiopathic lung fibroses, acute pulmonary fibroses, acute respiratory distress syndrome (ARDS), perimuscular fibroses, pericentral fibroses, dermatofibromas, kidney fibroses, diabetic nephropathy, glomerulonephrites, systemic or local scleroderma, keloids, hypertrophic sear formation, joint adhesions, arthroses, myelofibroses, cicatrization of the cornea, cystic fibrosis, muscular fibroses, Duchenne's muscular dystrophy, strictures of the esophagus, Ormond's disease, Crohn's disease, ulcerative colitis and aneurysms of the large vessels, as well as fibrotic diseases which are initiated or provoked by surgical scar revisions, plastic surgery, glaucoma, cataract fibroses, cicatrizations of the cornea, graft-versus-host disease, surgical interventions performed on tendons, nerve trapping syndromes, Dupuytren's contracture, adhesions resulting from gynecological interventions, pelvic adhesions, preidural fibroses, and diseases of the thyroid gland or the parathyroid glands, and also by metastatic bone invasion, by multiple mycloma or by restenoses.

## Revendications

**1.** Analogues de peptides de phosphinate de formule générale (II)

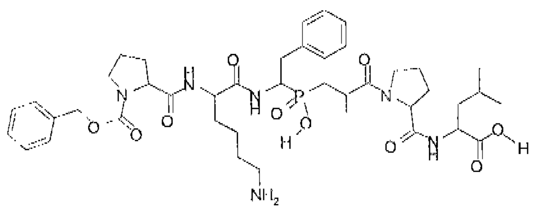

et/ou leurs stéréoisomères et sels.

**2.** Composés selon la revendication 1, **caractérisés en ce que** des composés de formule générale (II) présentant la configuration représentée dans la formule (Ib)

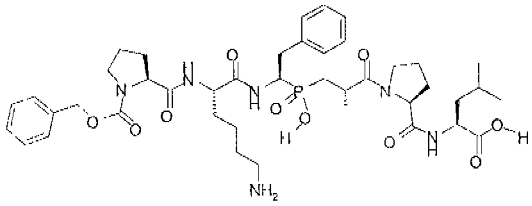

et/ou leurs sels sont sélectionnés.

3. Composés de formule (I)

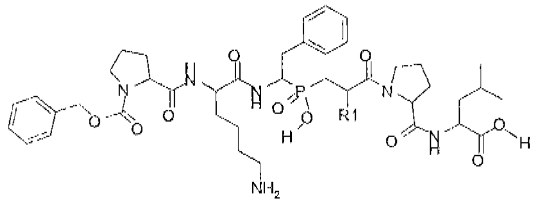

dans lesquels
R1 représente de l'hydrogène ou du méthyle,
et/ou leurs stéréoisomères et sels sont utilisés comme médicament destiné à la prophylaxie et/ou au traitement de maladies.

4. Médicament contenant au moins un composé de formule (I), tel que défini dans la revendication 3, et au moins un autre adjuvant.

5. Médicament contenant au moins un composé de formule (I), tel que défini dans la revendication 4, et au moins un autre agent.

6. Utilisation d'au moins un composé de formule (I), tel que défini dans la revendication 4, pour la fabrication d'un médicament destiné à la prophylaxie et/ou au traitement de maladies fibrotiques telles que, entre autres, les fibroses hépatiques d'origines diverses, comme par exemple la cirrhose du foie d'origine alcoolique, la cirrhose biliaire, les hépatites d'origine virale ou autre, les fibroses interstitielles idiopathiques, les fibroses pulmonaires idiopathiques, les fibroses pulmonaires aiguës, le «syndrome de détresse respiratoire aiguë» (ARDS), les fibroses périmusculaires, les fibroses pericentrales, les dermatofibromes, les fibroses néphrétiques, la néphiropathie diabétique, les glomérulonéphrites, la selérodermie systémique ou locale, les chéloïdes, la cicatrisation hypertrophique, les adhérences articulaires, les arthroses, les myélofibroses, les cicatrisations de la cornée, la fibrose cystique, les fibroses musculaires, la dystrophie musculaire de Duchenne, les rétrécissements de l'oesophage, la fibrose rétropéritonéale ou maladie d'Onnond, la maladie de Crohn, la colite ulcéreuse et les anévrismes des gros vaisseaux ainsi que les maladies fibrotiques qui sont initiées ou provoquées par des interventions chirurgicales de révision de cicatrice, la chirurgie plastique, les glaucomes, les fibroses de la cataracte, les cicatrisations de la cornée, la «réaction de greffe contre hôte» (GVIID), des interventions chirurgicales au niveau des tendons, les syndromes de compression nerveuse, la maladie de Dupuytren, les adhérences à la suite d'interventions gynécologiques, les adhérences pelviennes, les fibrose péridurales, les maladies de la glande thyroïde ou des glandes parathyroïdes, et par une affection osseuse métastatique, par le myélome multiple ou par des resténoses.

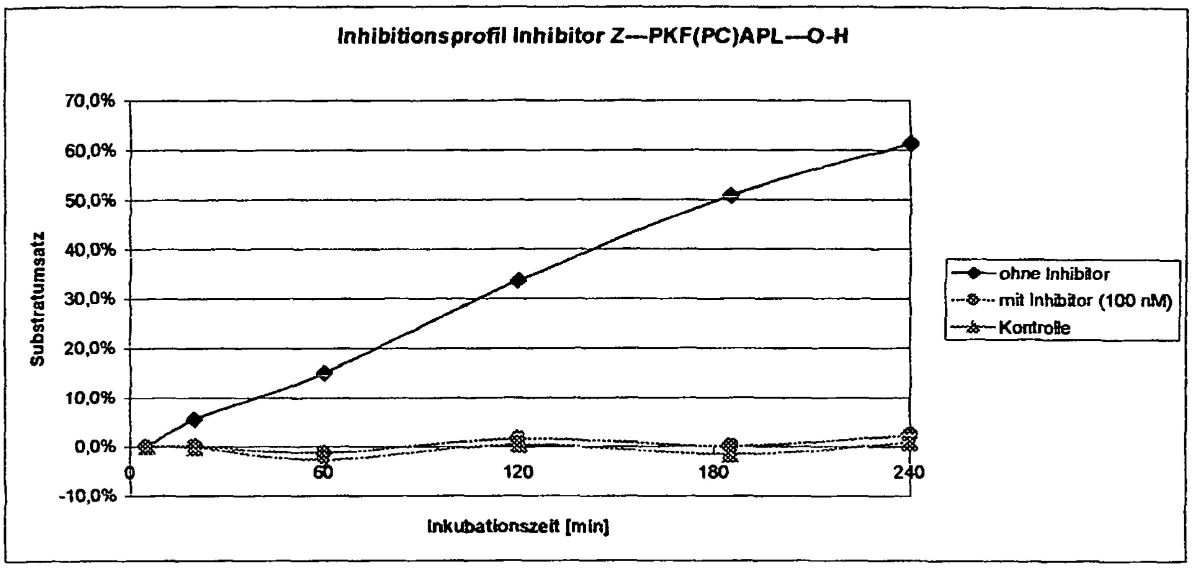
Inhibitionsprofil Inhibitor Z—PKF(PC)APL—O-H
70,0%
60,0%
50,0%
40,0%
30,0%
20,0%
10,0%
0,0%
-10,0%
Substratumsatz
0
60
120
180
240
Inkubationszeit [min]
ohne Inhibitor
mit Inhibitor (100 nM)
Kontrolle

Abbildung 1

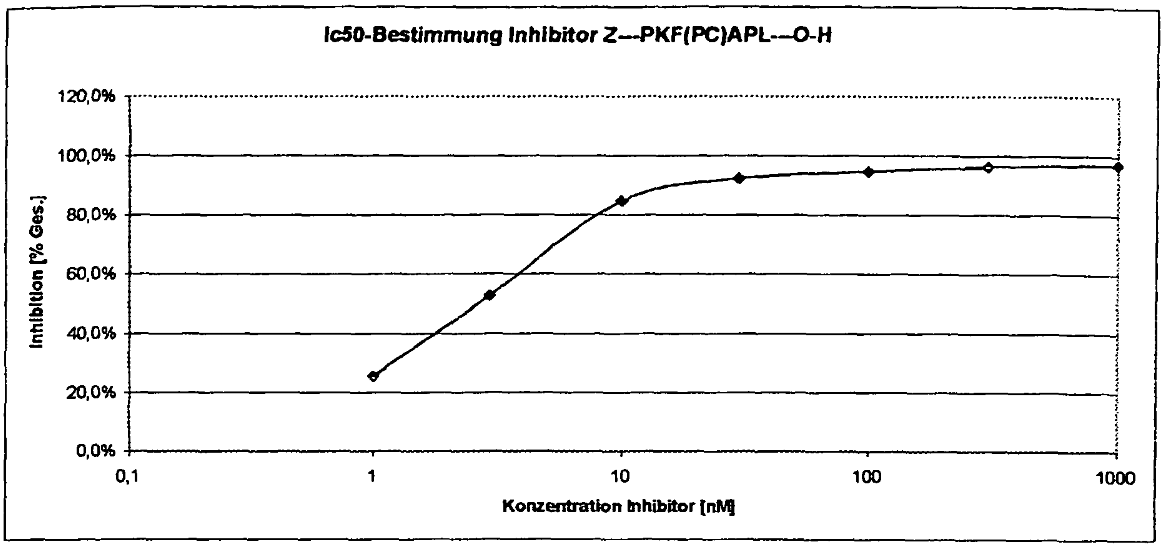
Ic50-Bestimmung Inhibitor Z---PKF(PC)APL---O-H
120,0%
100,0%
80,0%
60,0%
40,0%
20,0%
0,0%
Inhibition [% Ges.]
0,1
1
10
100
1000
Konzentration Inhibitor [nM]

Abbildung 2

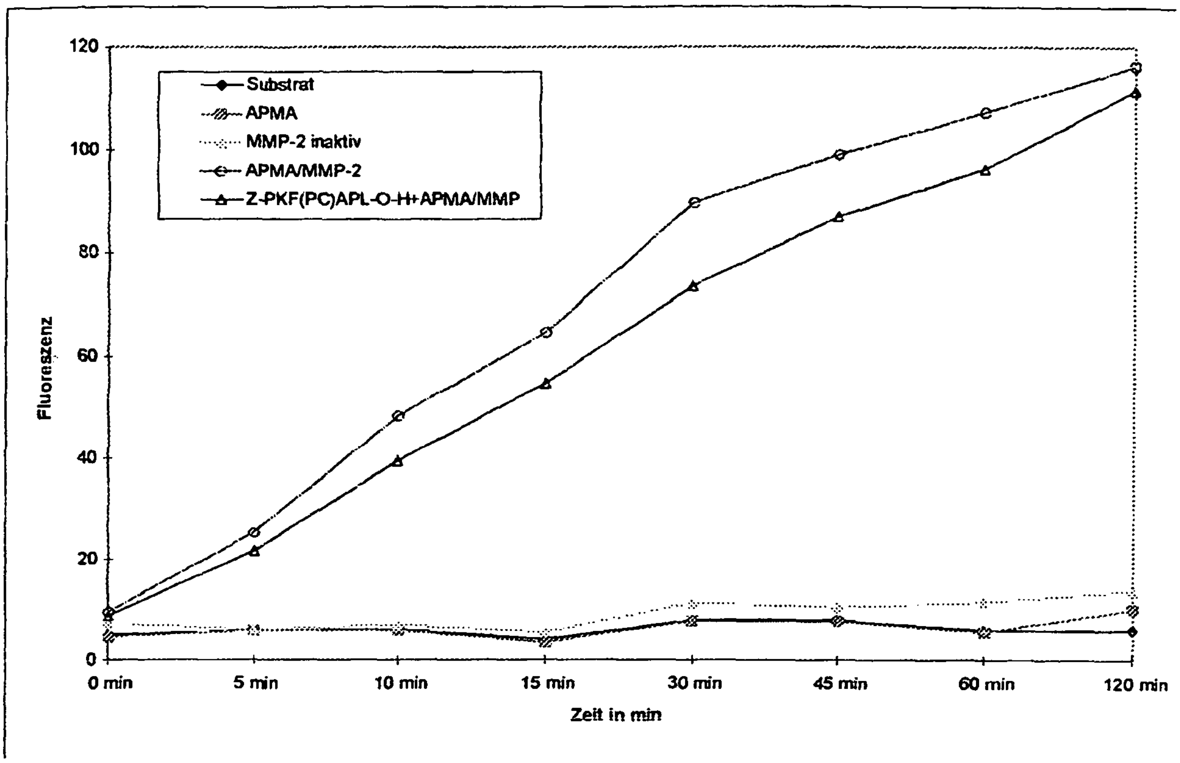
120
100
80
60
40
20
0
Fluoreszenz
Substrat
APMA
MMP-2 inaktiv
APMA/MMP-2
Z-PKF(PC)APL-O-H+APMA/MMP
0 min
5 min
10 min
15 min
30 min
45 min
60 min
120 min
Zeit in min

Abbildung 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9705865 A
- DE 19850072


### In der Beschreibung aufgeführte Nicht-Patentliteratur


- **AMANO S ; TAKAHARA K ; GERECKE D ; NISHIYAMA T ; LEE S ; GREENSPAN DS ; BURGESON RE.** Bone morphogenetic protein-1 is the processing enzyme for laminin 5 in human keratinocytes. *Mol. Biol. Cell,* 1996, vol. 7, 58A
- **OLSEN BJ.** Morphogenesis: collagen it takes and bone it makes. *Curr. Biol.,* 1996, vol. 6, 645-647
- **SUZUKI N ; LABOSKY PA ; FURATA Y ; HARGETT I ; DUNN R ; FOGO AB ; TAKAHARA K ; PETERS DM ; GREENSPAN DS ; HOGAN BL.** Failure of ventral body wall closure in mouse embryos lacking a procollagen C-proteinase encoded by BMP-1, a mammalian gene related to Drosophila tolloid. *Development,* 1996, vol. 122, 3587-3595
- **PACHENKO MV ; STETLER-STEVENSON WG ; TRUBETSKOY OV ; GACHERU SN ; KAGAN HM.** Metalloproteinase activity secreted by fibrogenic cells in the processing of prolyslyl oxidase. *Potential role of procollagen C-proteinase. J. Biol. Chem.,* 1996, vol. 2 (71), 7113-7119
- **MARQUES G ; MUSACCHIO M ; SHIMELL MJ ; WÜNNENBERG-STAPLETON K ; CHO KWY ; O'CONNOR MB.** Production of a DPP activity gradient in the early drosophila embryo through the opposing actions of the SOG and TLD proteins. *Cell,* 1997, vol. 91, 417-426
- **BLADER P ; RASTEGAR S ; FISCHER N ; STRÄHLE U.** Cleavage of the BMP antagonist chordin by zebrafish tolloid. *Science,* 1997, vol. 278, 1937-1940
- **KESSIER E ; TAKAHARA K ; BINIAMINOW L ; BRUSEL M ; GREENSPAN DS.** Bone morphogenetic protein-1. *The type I procollagen C-proteinase,* 1996
- **REDDI AH.** BMP-1: Resurrection as procollagen C-proteinase. *Science,* 1996, vol. 217, 463
- **LI SW ; SIERON AL ; FERTALA A ; HOJIMA Y ; ARNOLD WV ; PROCKOP DJ.** The C-proteinase that processes procollagens to fibrillar collagens is identical to the protein previosly identified as bone-morphogenetic protein-1. *Proc. Natl. Acad. Sci.,* 1996, vol. 93, 5127-5130
- **JANITZ M ; HEISER V ; BÖTTCHER U ; LANDT O ; LAUSTER R.** Three alternatively spliced variants of the gene coding for the human bone morphogenetic protein-1. *J. Mol. Med.,* 1998, vol. 76, 141-146
- **STÖCKER W ; GOMIS-RÜTH FX ; BODE W ; ZWILLING R.** Implications of the three-dimensional structure of astacin for the structure and function of the astacin family of zincendopeptidases. *Eur. J. Biochem.,* 1993, vol. 214, 215-231
- **STÖCKER W ; GRAMS F ; BAUMANN U ; REINEMER P ; GOMIS-RÜTH FX ; MCKAY DB ; BODE W.** The metzincins - Topological and sequential relations between the astacins, adamalysins, serralysins, and matrixins (collagenases) define a superfamily of zincendopeptidases. *Prot. Sci.,* 1995, 823-840
- **GRAMS F ; DIVE V ; YIOTAKIS A ; YIALLOUROS I ; VASSILOU S ; ZWILLING R ; BODE W ; STÖCKER W.** Structure of astcin with transition-state analogue inhibitor. *Nature Struct. Biol.,* 1996, vol. 3, 671-675
- **YIOTAKIS A ; VASSILIO S ; JIRACEK J ; DIVE V.** Protection of the Hydroxyphosphinyl Function of-Phosphinic Dipeptides by Adamantyl. Application to the Solid-Phase Synthesis of Phosphinic Peptides. *J. Org. Chem.,* 1996, vol. 61, 6601-6605
- **CAMPAGNE JM ; COSTE J ; GUILLOU L ; HEITZ A ; JOUIN P.** Solid phase synthesis of phosphinic peptides. *Tetrahedron Lett.,* 1993, vol. 34, 4181-4184
- **YIALLOUROS I ; VASSILIOU S ; YIOTAKIS A ; ZWILLING R ; STÖCKER W ; DIVE V.** Phosphinic peptides, the first potent inhibitors of astacin, behave as extremely slow-binding inhibitors. *Biochem. J.,* 1998, vol. 331, 375-379
- **LEE ST ; KESSLER E ; GREENSPAN DS.** Analysis of site-directed mutations in human pro-a2(I) collagen which block cleavage by the C-proteinase. *J. Biol. Chem.,* 1990, vol. 265, 21992-21996
- **MATAYOSHI ED ; WANG GT ; KRAFFT GA ; ERICKSON J.** Novel fluorogenic substrates for assaying retroviral proteases by resonance energy transfer. *Science,* 1989, vol. 247, 954-958

- **JM, ROSEN V ; CELESTE AJ ; MITSOCK LM ; WHITTERS MJ ; KRIZ RW ; HEWICK RM ; WANG EA.** Novel regulators ofbone formation: molecular clones and activities. *Science,* 1988, vol. 242, 1528-1534
- Instruction manual strep tag II System. *Biometra,* 1994
- Molecular Cloning. **SAMBROOK J ; FRITSCH EF ; MANIATIS T.** A Laboratory Manual. Cold Spring Harbor Laboratory Press, 1989
- **PROCKOP DJ ; SIERON AL ; LI SW.** Procollagen N-proteinase and procollagen C-proteinase. Two unusual metalloproteinases that are essential for procollagen processing probably have important roles in development and cell signaling. *Matrix Biol.,* 1998, vol. 16, 399-408
- **AIMES, RT ; QUIGLEY JP.** Matrix Metalloptotenase-2 is an interstitial Collagenase-Inhibitor-free enzyme catalyzes the cleavage of collagen fibrils and soluble native type-I collagen generating the specific ¾-length and ¼-length fragments. *J. Biol. Chem.,* 1995, vol. 270, 5872-5876
- **GREGORY A. GRANT ; ARTHUR Z. EISEB ; BARRY L. MARMER ; WILLIAM T. ROSWIT ; GREGORY I. GOLDBERG.** The Activation of Human Fibroblast Procollagenase. *J. Biol. Chem.,* 1987, vol. 262, 5886-5889
- **BICKETT DM ; GREEN MD ; BERMAN J ; DEZUBE M ; HOWE AS ; BROWN PJ ; ROTH JT ; MCGEEHAN GM.** A high throughput fuorogenic substrate for interstitial collagenase (MMP-1) and gelatinase (MMP-9. *Anal. Biochem.,* 1993, vol. 212, 58-64
- **BURCHARDT ER ; SCHRÖDER W ; HEKE M ; KOHLMEYER J ; NEUMANN R ; KROLL W.** Expression cloning of C-terminal procollagen (III) propeptide and its use in a novel serum assay to monitor liver fibrogenesis. *Hepatology,* 1997, vol. 26, 487A
- **JOHNSON S J ; HINES JE ; BURT AD.** Phenotypic modulation of perisinusoidal cells following acute liver injury: a quantitative analysis. *Int. J. Exp. Path.,* 1992, vol. 73, 765-772
- **MCLEAN E ; MCLEAN A ; SUTTON P.** Instant Cirrhosis. An improved method for producing cirrhosis of the liver in rats by simultaneous administration of carbon tetrachloride and phenobarbitone. *Br. J. Exp. Pathol.,* 1969, vol. 50, 502-506
- **KOUNTOURAS J ; BILLING B ; SCHEUER P.** Prolonged bile obstruction: a new experimental model for cirrhosis in the rat. *Br. J. Exp. Pathol.,* 1984, vol. 65, 305-311
- **BHUNCHET E ; WAKE K.** Suppression of experimental hepatic fibrosis by administration of vitamin. *A.Lab. Invest.,* 1985, vol. 52, 182-194
- **GERLING B ; BECKER M ; WALDSCHMIDT J ; REHMANN M ; SCHUPPAN D.** Elevated serum aminoterminal procollagen type-III-peptide parallels collagen accumulation in rats with secondary biliary fibrosis. *Hepatology,* 1996, vol. 25, 79-84
- **KAUSCHKE SG ; KNORR A ; OLZEN M ; BURCHARDT ER.** Expression of collagen (III) as determined by quantitative PCR and its correlation with extracellular collagen deposition in the rat CCl4 model of liver fibrosis. *Hepatology,* 1997, vol. 26, 538A